(19) Europäisches Patentamt
European Patent Office
Office européen des brevets

(11) EP 2 589 669 B1

(12) EUROPEAN PATENT SPECIFICATION

(45) Date of publication and mention of the grant of the patent:
22.10.2014 Bulletin 2014/43

(51) Int Cl.:
*C12Q 1/68* (2006.01)

(21) Application number: 13152518.0

(22) Date of filing: 11.04.2011

(54) **Single nucleotide polymorphisms that predict HCV treatment outcomes**

EINZELNUCLEOTIDPOLIMORPHISMUS ZUR VORHERSAGE DES ERGEBNISSES EINER BEHANDLUNG BEI HCV

POLYMORPHISME DE NUCLÉOTIDE UNIQUE POUR LA PRÉDICTION DU RÉSULTAT D'UN TRAITEMENT DU VHC

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 13.04.2010 US 323502 P

(43) Date of publication of application:
08.05.2013 Bulletin 2013/19

(62) Document number(s) of the earlier application(s) in accordance with Art. 76 EPC:
11717518.2 / 2 558 592

(73) Proprietor: F. Hoffmann-La Roche AG
4070 Basel (CH)

(72) Inventors:
• Chu, Tom
  Mont Clair, NJ 07042 (US)
• Essioux, Laurent
  68220 Attenschwiller (FR)
• Germer, Soren
  Maplewood, NJ 07040 (US)
• Lopatin, Uri
  San Francisco, CA 94103 (US)
• Shulman, Nancy
  Palo Alto, CA 94303 (US)
• Thommes, James
  San Francisco, CA 94117 (US)

(74) Representative: Halbig, Dirk
F. Hoffmann-La Roche AG
Patent Department
Grenzacherstrasse 124
4070 Basel (CH)

(56) References cited:
WO-A2-2009/046369     US-A1- 2005 014 159
US-A1- 2005 260 567

• RAUCH ANDRI ET AL: "Genetic variation in IL28B is associated with chronic hepatitis C and treatment failure: a genome-wide association study", GASTROENTEROLOGY, ELSEVIER, PHILADELPHIA, PA, vol. 138, no. 4, 1 April 2010 (2010-04-01), pages 1338-1345,13, XP002601554, ISSN: 0016-5085, DOI: DOI:10.1053/J.GASTRO. 2009.12.056
• TANAKA YASUHITO ET AL: "Genome-wide association of IL28B with response to pegylated interferon-alpha and ribavirin therapy for chronic hepatitis C", NATURE GENETICS, NATURE PUBLISHING GROUP, US, vol. 41, no. 10, 1 October 2009 (2009-10-01), pages 1105-1109, XP002602520, ISSN: 1546-1718, DOI: DOI: 10.1038/NG.449 [retrieved on 2009-09-13]
• SUPPIAH VIJAYAPRAKASH ET AL: "IL28B is associated with response to chronic hepatitis C interferon-alpha and ribavirin therapy", NATURE GENETICS, NATURE PUBLISHING GROUP, US, vol. 41, no. 10, 1 October 2009 (2009-10-01), pages 1100-1104, XP002601552, ISSN: 1546-1718, DOI: DOI:10.1038/NG.447 [retrieved on 2009-09-13]

- YEN Y-H ET AL: "Mutations in the interferon sensitivity-determining region (nonstructural 5A amino acid 2209-2248) in patients with hepatitis C-1b infection and correlating response to combined therapy of pegylated interferon and ribavirin", ALIMENTARY PHARMACOLOGY & THERAPEUTICS, BLACKWELL SCIENTIFIC PUBLICATIONS LTD., CAMBRIDGE, GB, vol. 27, no. 1, 1 January 2008 (2008-01-01), pages 72-79, XP002582304, ISSN: 0269-2813, DOI: DOI: 10.1111/J.1365-2036.2007.03560.X [retrieved on 2007-10-25]

**Description**

The present invention relates to methods that useful for predicting the response of hepatitis C virus (HCV) infected patients to pharmacological treatment.

Background of the invention

The standard of care for chronic hepatitis C is the combination of peginterferon plus ribavirin.[1] Overall sustained virological response (SVR) rates after treatment with the standard of care are approximately 50%,[2-4] although it is difficult to predict whether an individual patient will achieve an SVR.

The probability of achieving an SVR varies with a collection of patient and viral factors. For example, younger patients, Caucasian and Asian patients, and individuals without advanced hepatic fibrosis are more likely to clear HCV infection after treatment.[5-8] Similarly, patients infected with HCV genotypes 2 or 3, rather than genotype 1, and those with low baseline HCV RNA levels in serum have the best chance of a cure.[2-4, 6, 8]

More precise prediction of SVR is currently possible only after the start of treatment. Regardless of HCV genotype, individuals who clear HCV RNA after 4 or 12 weeks of treatment have a much better chance of achieving an SVR than those with persistent viremia.[9] Rapid virological response (RVR, undetectable HCV RNA at week 4) is a strong predictor of SVR; conversely, failure to achieve an early virological response (EVR, greater than a two log decline in HCV RNA at week 12) is a strong predictor of nonresponse, independent of pretreatment characteristics.[10]

The ability to prospectively differentiate between potential responders and non-responders to the standard of care could have a great impact on the care of patients with chronic hepatitis C. Treatment decisions could be personalized based on the likelihood of patients to respond to the standard of care. For example patients with the lowest likelihood of achieving an SVR with the current standard of care might defer treatment until direct acting antiviral agents are available. Conversely, patients with a high likelihood of achieving an SVR might prefer to initiate therapy immediately with a treatment regimen that is a known entity.

In addition to host and viral factors, host genetic diversity also influences the response to treatment with the standard of care.[11] Recent evidence from genome-wide association studies suggests that single nucleotide polymorphisms (SNPs) in the promoter region of the *IL-28b* gene exert a strong influence on the probability of SVR in patients treated with peginterferon plus ribavirin.[12-14] The objectives of this analysis were to explore SNPs associated with both early virological response (EVR) and SVR in a diverse cohort of patients that includes treatment-naive individuals and nonresponders to a previous course of peginterferon alfa-2b (12KD) plus ribavirin who received peginterferon alfa-2a (40KD) monotherapy or combination therapy with either peginterferon alfa-2a (40KD) or conventional interferon plus ribavirin.

Reference RAUCH ANDRI ET AL: "Genetic variation in IL28B is associated with chronic hepatitis C and treatment failure: a genome-wide association study", GASTROENTEROLOGY, ELSEVIER; PHILADELPHIA, PA, vol. 138, no. 4, 1 April 2010 (2010-04-01), pages 1338 - 1345 reports a genome-wide association study to screen for sustained response to pegylated interferon alfa and ribavirin therapy. The study identified e.g. the SNP rs8099917 to influence natural and treatment induced control of HCV infection.

Summary of the invention

The present invention is based on the discovery of an association between the SNP rs 10023606 on human chromosome four and SVR in patients treated with interferon-based regimens. In one embodiment, the invention provides for a method for predicting sustained virological response of a human subject infected with HCV to interferon treatment comprising, providing a sample from said human subject, detecting the presence of the single nucleotide polymorphism within chromosome 4 and determining that said subject has a high likelihood of sustained virological response to interferon treatment if said single nucleotide polymorphism is present, wherein said single nucleotide polymorphism is a G at rs10023606.

Brief description of the drawings

Figure 1. Genome-wide association results for (a) SVR (b) EVR) and (c) SVR after adjustment for rs 12979860 by chromosome in the overall genotype 1 population.

Figure 2. Quantile-quantile plot of the distribution of test statistics for (a) SVR and (b) EVR). Grey circles denote expected p-values and blue circles show observed p values.

Figure 3. Linkage disequilibrium of significant SNPs (p<10^-5) in the IL-28 region of the Caucasian genotype 1 population.

Detailed description of the invention

Definitions

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The term "response" to treatment with interferon is a desirable response to the administration of an agent. Virological endpoints included "early virological response" (EVR), defined as ≥2-log drop in serum HCV RNA from baseline to week 12 (by Cobas Amplicor HCV Monitor Test, v2.0, limit of quantitation 600 IU/mL), complete EVR (cEVR) defined as undetectable HCV RNA in serum (by Cobas Amplicor HCV Test v2.0, limit of detection 50 IU/mL) or and "sustained virological response" (SVR), defined as undetectable HCV RNA (<50 IU/mL) at the end of a 24-week untreated follow-up period.

The terms "sample" or "biological sample" refers to a sample of tissue or fluid isolated from an individual, including, but not limited to, for example, tissue biopsy, plasma, serum, whole blood, spinal fluid, lymph fluid, the external sections of the skin, respiratory, intestinal and genitourinary tracts, tears, saliva, milk, blood cells, tumors, organs. Also included are samples of in vitro cell culture constituents (including, but not limited to, conditioned medium resulting from the growth of cells in culture medium, putatively virally infected cells, recombinant cells, and cell components).

The terms "interferon" and "interferon-alpha" are used herein interchangeably and refer to the family of highly homologous species-specific proteins that inhibit viral replication and cellular proliferation and modulate immune response. Typical suitable interferons include, but are not limited to, recombinant interferon alpha-2b such as Intron® A interferon available from Schering Corporation, Kenilworth, N.J., recombinant interferon alpha-2a such as Roferon®-A interferon available from Hoffmann-La Roche, Nutley, N.J., recombinant interferon alpha-2C such as Berofor® alpha 2 interferon available from Boehringer Ingelheim Pharmaceutical, Inc., Ridgefield, Conn., interferon alpha-n1, a purified blend of natural alpha interferons such as Sumiferon® available from Sumitomo, Japan or as Wellferon® interferon alpha-n1 (INS) available from the Glaxo-Wellcome Ltd., London, Great Britain, or a consensus alpha interferon such as those described in U.S. Pat. Nos. 4,897,471 and 4,695,623 (especially Examples 7, 8 or 9 thereof) and the specific product available from Amgen, Inc., Newbury Park, Calif., or interferon alpha-n3 a mixture of natural alpha interferons made by Interferon Sciences and available from the Purdue Frederick Co., Norwalk, Conn., under the Alferon Tradename. The use of interferon alpha-2a or alpha-2b is preferred. Interferons can include pegylated interferons as defined below.

The terms "pegylated interferon", "pegylated interferon alpha" and "peginterferon" are used herein interchangeably and means polyethylene glycol modified conjugates of interferon alpha, preferably interferon alfa-2a and alfa-2b. Typical suitable pegylated interferon alpha include, but are not limited to, Pegasys® and Peg-Intron®.

The term "ribavirin" refers to the compound, 1-((2R,3R,4S,5R)-3,4-Dihydroxy-5-hydroxymethyl-tetrahydro-furan-2-yl)-1H-[1,2,4]triazole-3-carboxylic acid amide which is a synthetic, non-interferon-inducing, broad spectrum antiviral nucleoside analog and available under the names, Virazole® and Copegus®.

"Direct acting antiviral agents" exert specific antiviral effects independent of immune function. Examples of direct acting antiviral agents for HCV include but are limited to protease inhibitors, polymerase inhibitors, NS5A inhibitors, IRES inhibitors and helicase inhibitors.

The current recommended first line treatment for patients with chronic hepatitis C is pegylated interferon alpha in combination with ribavirin for 48 weeks in patients carrying genotype 1 or 4 virus and for 24 weeks in patients carrying genotype 2 or 3 virus. Combined treatment with ribavirin was found to be more effective than interferon alpha monotherapy in patients who relapsed after one or more courses of interferon alpha therapy, as well as in previously untreated patients. However, ribavirin exhibits significant side effects including teratogenicity and carcinogenicity. Furthermore, ribavirin causes hemolytic anemia requiring dose reduction or discontinuation of ribavirin therapy in approximately 10 to 20% of patients, which may be related to the accumulation of ribavirin triphosphate in erythrocytes. Therefore, to reduce treatment cost and the incidence of adverse events, it is desirable to tailor the treatment to a shorter duration while not compromising efficacy. A shortened "duration of treatment" for genotype 1 patients with pegylated interferon alpha with ribovirin would be, for example, 24 weeks. A shortened duration of treatment for genotype 1 patients with pegylated interferon alpha with ribavirin in combination with a direct acting antiviral agent could be as short as 8 weeks, 12 weeks, or 16 weeks.

As used herein, the terms "allele" and "allelic variant" refer to alternative forms of a gene including introns, exons, intron/exon junctions and 3' and/or 5' untranslated regions that are associated with a gene or portions thereof. Generally, alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides.

As used herein, the term "polymorphism" refers to the coexistence of more than one form of a nucleic acid, including exons and introns, or portion (e.g., allelic variant) thereof. A portion of a gene of which there are at least two different forms, i.e., two different nucleotide sequences, is referred to as a polymorphic region of a gene. A polymorphic region can be a single nucleotide, i.e. "single nucleotide polymorphism" or "SNP", the identity of which differs in different alleles. A polymorphic region can also be several nucleotides long.

Numerous methods for the detection of polymorphisms are known and may be used in conjunction with the present invention. Generally, these include the identification of one or more mutations in the underlying nucleic acid sequence either directly (e.g., in situ hybridization) or indirectly (identifying changes to a secondary molecule, e.g., protein sequence or protein binding).

One well-known method for detecting polymorphisms is allele specific hybridization using probes overlapping the mutation or polymorphic site and having about 5, 10, 20, 25, or 30 nucleotides around the mutation or polymorphic region. For use in a kit, e.g., several probes capable of hybridizing specifically to allelic variants, such as single nucleotide polymorphisms, are provided for the user or even attached to a solid phase support, e.g., a bead or chip.

The single nucleotide polymorphism, "rs12979860" refers to a SNP identified by its accession number in the database of SNPs (dbSNP, www.ncbi.nlm.nih.gov/SNP) and is located on human chromosome 19 in the promoter region of the IL28b gene.

The results of this analysis of data from two large multinational studies (described in the Examples section) confirm and extend the remarkable association between SNPs in the IL-28 region and SVR after treatment with peginterferon plus ribavirin that was first reported by Ge et al.[12] In the present analysis, the top SNP (rs1297980) was associated with both EVR (p=5.0 x 10$^{-26}$) and SVR (p=1.4 x 10$^{-21}$). The inclusion of patients who achieved an SVR after treatment not only with peginterferon alfa-2a (40KD) plus ribavirin, but also with peginterferon alfa-2a (40KD) monotherapy and the combination of conventional interferon plus ribavirin, as well as the well-pedigreed nature of the large subgroup of non-responders are unique strengths of the study.

This study allows for the generation of several unique suggestions. First, we suggest that the effect of rs12979860 is exerted most likely on EVR rather than on SVR. An association with EVR, but not SVR, was seen in previous non-responders to therapy and in patients treated with peginterferon alfa-2a (40KD) monotherapy, or conventional interferon plus ribavirin. We interpret this to mean that the mechanism through which this SNP acts is associated with the rapid first-phase decline in serum HCV RNA levels rather than with the slower second phase decline or hepatocyte turnover. The first phase decline in HCV RNA is a well described phenomenon that occurs shortly after the initiation of interferon-based therapy and has been suggested to be an indicator of a particular patient's sensitivity to interferon.[19]

Next, the particular chip used in this study allowed us to rank the significance of a series of SNPs in addition to rs12979860, all of which were identified in previous studies.[12-14, 20, 21] Although several highly significant markers were identified in the IL28b region, none of them were significantly associated with SVR after adjustment for rs1297980. Only one SNP (rs8099917) was statistically significantly associated with EVR after adjustment for rs12979860; however, the association was such that it would not have been deemed significant after adjustment for multiple comparisons.

In addition, by adjusting for rs12979860 we were able to begin exploring potential gene-gene interactions that may affect the probability of achieving a virological response. As noted above, none of the other highly significant SNPs in the IL28b region identified in the primary analysis were retained after this process; however, a series of previously unreported SNPs located on chromosome 4 were revealed.

Consistent results obtained in several retrospective studies[12-14,20-23] including this one, allow us to say with confidence that SNPs in the promoter region of IL28b are highly associated with the outcome of treatment with interferon-based therapy. It has been suggested that changes in one of the multiple promoters in this region alter hepatic expression of interferon lambda. Interferon lambda is a type III interferon that triggers a signalling pathway that overlaps with the Jak/Stat pathway of type I interferons (including interferon-alpha).[24-26] We hypothesise that in patients with a "permissive" SNP, HCV infection induces local production of interferons -including type III interferons (such as IL28). This results in interferon-induced gene expression in the liver. This hypothesis is consistent with prior reports that non-responders to interferon-based therapy have increased hepatic expression of interferon-stimulated genes (ISGs) in their liver prior to the initiation of therapy, and are in an apparent "pre-activated" state.[27] This apparent paradoxical relationship between the level of endogenous ISG induction and the subsequent responsiveness to interferon-based therapy has not been clearly explained. Some investigators have speculated that some of the downstream effects of ISG induction on an axis similar to protein kinase PKR might suppress endogenous protein production via phosphorylation of eukaryotic initiation factor 2 alpha (eIF2a) and result in hepatocytes that, being refractory to further stimulation by exogenous interferon, are unable to further attenuate protein production.[28] HCV RNA is translated via an internal ribosomal entry site and is partially eIF2a- independent. Thus, replication of HCV RNA and assembly of further virions can continue unabated despite the "pre-activated" state.[28] This hypothesis explains why a poorly responsive GWAS genotype is associated with a lower viral load than the more treatable genotype in our analysis, in spite of previous analyses that suggest increasing baseline viral load is an indicator of poor treatment outcomes.[6] Endogenous production of interferon partially impairs viral replication, but simultaneously impairs host cell protein synthesis in these patients. There may be a threshold above which host cells cannot further decrease protein production or increase stimulation of ISGs. Thus, exogenous interferon cannot augment the host response. Paradoxically, administration of exogenous pegylated interferon can, however, "rescue" patients who are less sensitive to endogenous interferon. The genetic polymorphism may play a role in ISG expression. This leads us to speculate that it may eventually be possible to selectively target the interferon pathway to produce a more favourable ISG profile that results in HCV eradication.

It is more challenging to explain the possible mechanism by which a SNP in chromosome 4 is associated with SVR, though not with EVR. The simplest explanation might require a gene in this region to be involved in a phenomenon that is exclusively associated with SVR such as hepatocyte turnover. Additional host-host or host-viral gene interactions may be involved in the mechanism by which both loci exert their effects, and require further careful evaluation.

The discovery of an interferon treatment susceptibility locus in the *IL28b* region has implications for therapy with the current standard of care (SOC), i.e. peginterferon plus ribavirin. In the near future, one might envisage that in some patients the *IL28b* genotype will be determined before therapy is initiated and that patients will be stratified not only by HCV genotype - as currently recommended[1] - but also by human genotype. In contrast to the current treatment paradigm, the initial treatment regimen will be based upon both viral and human genotype. The putative role of ribavirin in this context will need to be addressed in prospective trials.

This discovery also has implications for the ongoing development programs of direct acting antiviral agents for HCV therapy. Direct acting antiviral agents exert specific antiviral effects independent of immune function, but it is thought that at least some of the drug classes in development may benefit from an additive (if not synergistic) innate immunomodulatory effect. Indeed it is likely that interferon-based therapy will remain the backbone of the treatment because it is needed to prevent the emergence of resistant HCV.[29] HCV RNA encodes specific proteins that may inhibit the induction of type I interferons. For example the NS3-4A protease of HCV blocks dsRNA-induced interferon production by interfering with phosphorylation of interferon regulatory factor-3 (IRF-3).[30] Thus, the NS3-4A protease is a dual therapeutic target, whose inhibition may block viral replication and restore IRF-3 control of HCV RNA replication. Protease inhibitors such as Telaprevir, which have robust antiviral effects when administered in combination with a second small molecule or the standard of care, also inhibit the protease functions by which HCV impairs host interferon response. It will be important to observe whether treatment outcomes are similar when direct acting antiviral agents are combined with peginterferon plus ribavirin in patients with interferon-refractory and interferon-sensitive IL28b phenotypes. It is possible that interferon-refractory patients will respond to triple therapy (direct acting antiviral agent plus peginterferon plus ribavirin) as if they were on monotherapy with the direct acting antiviral agent alone. If this is the case then patients with the interferon-refractory IL28b phenotype may be much more susceptible to the selection of resistance mutations during treatment with a drug such as telaprevir.[31] These possibilities suggest scenarios where patients with the interferon-susceptible SNP (rs12979860) might benefit from abbreviated treatment with peginterferon and ribavirin, and those with an interferon-refractory genotype might be candidates for extended treatment durations and/or more intensive treatment regimens.. Alternatively, interferon-free combination regimens of direct acting antiviral agents may be more appropriate for patients with an interferon-refractory genotype.

Our unique dataset, including a registration trial of peginterferon alfa-2a plus ribavirin therapy for patients who were previous non-responders to standard of care with a pegylated interferon, highlights the correlation of the rs12979860 SNP with EVR rather than SVR. Stated differently, this SNP defines patient's responsiveness to interferon rather than the ultimate response to therapy and thus helps identify patients likely or unlikely to undergo an SVR because of their likelihood to achieve an EVR. It is unlikely that it predicts SVR independently of EVR. This has tremendous implications for use of direct acting antiviral agents together with interferon.

Utilization of baseline genetic predictors for interferon responsiveness allows tailoring of direct acting antiviral therapies, as well as standard of care with interferon. Patients who are defined as poorly interferon responsive, i.e. two T alleles in rs12979860, may be poor candidates for small molecule therapeutics that rely on additive or synergistic effects of endogenous or exogenous interferon mediated pathways - particularly if these are provided as a single agent in addition to SOC. The concern would be that these patients would have an increased risk of developing drug resistant mutations as a result of effective monotherapy.

Conversely, patients with an interferon responsive phenotype, i.e. two C alleles in rs12979860, would make superior candidates for shorter courses of therapy with SOC alone, or in combination with small molecules. Additional considerations that genetic predisposition to interferon responsiveness allow is "tuning" of combination direct acting antiviral agents. Patients predicted to have acceptable endogenous interferon responsiveness may be excellent candidates for drugs that target viral functions-such as protease inhibitors which also have an inhibitory role on endogenous interferon response- and poorer candidates for drugs that decrease the amounts of viral PAMP (pathogen associated molecular pattern, e.g. polymerase inhibitors) as these may serve to impair the patients capacity to facilitate their own cure via their endogenous interferon responsiveness. Similarly, patients predicted to have poor interferon responsiveness might be candidates for "quad" therapy as a first line of therapy (2 direct acting antiviral agents, added to peginterferon with ribavirin), as compared to a direct acting antiviral agent alone, or triple therapy (SOC with one DAA).

Examples

Methods

Patients Sample collection and virological end-points

Samples were analysed from a subset of patients with chronic hepatitis C enrolled in 2 large, randomised, multinational, phase III trials.[3, 15] In one study interferon-naive patients were randomised to 48 weeks of treatment with peginterferon alfa-2a (40KD) alone or in combination with ribavirin, or conventional interferon alfa-2b plus ribavirin.[3] Only non-responders to a previous 12-week course of peginterferon alfa-2b (12KD) plus ribavirin were eligible for the second trial, which randomised patients to either 48 or 72 weeks of treatment with either a standard or induction dose regimen of peginterferon alfa-2a (40KD) (all patients received standard dose ribavirin).[15] The study design, inclusion and exclusion criteria and primary results of these trials are published elsewhere.[3, 15]

Blood samples collected from patients who consented to participate in genetic analyses were stored in the Roche Clinical Sample repository. DNA was extracted at the Roche Clinical Sample repository and normalized to 50 ng/$\mu$L. Samples initially underwent a quality check using a Y-chromosome specific TaqMan Assay (Applied Biosystems, Foster City, CA) to assess both DNA quality and gender concordance with clinical data.

Virological endpoints included early virological response (EVR), defined as undetectable HCV RNA in serum (by Cobas Amplicor HCV Test v2.0, limit of detection 50 IU/mL) or $\geq$2-log drop in serum HCV RNA from baseline to week 12 (by Cobas Amplicor HCV Monitor Test, v2.0, limit of quantitation 600 IU/mL) and sustained virological response (SVR), defined as undetectable HCV RNA (<50 IU/mL) at the end of a 24-week untreated follow-up period.

In the GWAS analysis for SVR, the responder group was composed of all genotype-1 patients with SVR from the interferon naive study population. Non-responders were composed of 1) all genotype-1 non-SVR patients from the study population re-challenged by pegylated-Interferon treatment, 2) genotype-1 non-SVR patients form the interferon naive study population treated with pegylated interferon with ribavirin.

All responders from the study population re-challenged by the pegylated interferon population were therefore excluded from our analysis.

In the GWAS analysis for EVR, the EVR group was composed of all genotype-1 patients with EVR from the interferon naive study population. The non-EVR group was composed of 1)all patients from the patients population re-challenged by pegylated-Interferon and 2) by the genotype -1 non EVR patients treated with pegylated interferon + ribavirin from the treatment naive study population.

Additional exploratory analysis were conducted in the IL28B region SNP the Self-reported Caucasian in non-genotype 1 patients, and in each trial separately. (see details in the result section).

Genotypic data analysis

Samples were genotyped for 1,016,423 markers on the Illumina Infinium® HD Assay Super, using HumanOmniI Quad (v1.0) chips and iScan scanner. Initial quality control was performed to zero-out SNPs for discordant calls between replicate samples, excess heterozygosity, low call rate (<0.95), cluster separation, GenTrain score, intensities and cluster width. A total of 25 samples failed initial quality checks or subsequent genotyping attempts. After quality control, 1,002,139 SNPs with genotype calls were generated. The number of SNPs per chromosome, distribution of allelic frequencies and Hardy Weinberg equilibrium were calculated for the self-reported Caucasian population.

Statistical analysis

Associations between virological response (EVR or SVR) and baseline variables (age, body mass index [BMI], HCV RNA level and ALT quotient, entered as continuous variables, and sex, HCV genotype, histological diagnosis [presence or absence of cirrhosis] and race entered as categorical variables) were evaluated in a univariate logistic regression model.

Logistic regression (PROC LOGISTIC, SAS v9.2) was used to test for associations between individual SNPs and response/non-response after adjustment for baseline BMI, sex, age, viral load, ALT quotient and principal components analysis (PCA) components.

The analysis of ancestry was based on PCA as proposed by Purcell et al.[16] using an ancestry dataset created using the overall population ("pgt"). The overall population was supplemented with HapMap Phase III founders from 11 ethnically diverse subject sets and analysed in SAS JMP Genomics (SAS Institute Inc., Cary, NC, USA). PCA components were compared with and without outliers. Outliers were defined as individuals whose ancestry was at least 6 standard deviations from the mean on one of the top ten inferred axes of variation.

SNPs that were on the X and Y chromosomes or in mitochondrial DNA were removed along with SNPs that were not genotyped in HapMap phase III founders (release number 27), or were in regions with known high linkage disequilibrium (Chr5, 44-51.5Mb; Chr6, 24-36Mb; Chr8, 8-12Mb; Chr11, 42-58Mb; Chr17, 40-43Mb). Remaining SNPs were thinned using PLINK[16] with a window size of 1000, $r^2 < 0.25$ and a window shift of 100.

The association between virological response (EVR and SVR) and individual SNPs was tested by logistic regression analysis. Adjustments were made for baseline characteristics (BMI, sex, age, baseline HCV RNA level, ALT quotient and PCA components). Significance was assessed by likelihood ratio test (LRT).

The null model was defined as follows:

$$\text{Virological response} = \text{BMI} + \text{sex} + \text{age} + \text{HCV RNA level} + \text{ALT quotient} + \text{PCA components}.$$

In the null model the genetic effect of SNPs was assumed to be zero.

The alternative model was defined as:

$$\text{Virological response} = \text{BMI} + \text{sex} + \text{age} + \text{HCV RNA level} + \text{ALT quotient} + \text{PCA components} + \text{SNP}$$

Maximum likelihood estimates for each alternative model (i.e. with SNPs) were compared to the corresponding null model (i.e., without SNPs) using LRT, which has a chi-square distribution and degrees of freedom equal to the number of differing parameters. SNPs were entered into the model as continuous variables (0, 1, 2). PCA components represented the top five components in the analysis populations. Similar models were run in the self-reported Caucasian population and in the non-genotype 1 population.

Linkage disequilibrium (LD) was calculated among significant SNPs ($p<10^{-5}$) in the *IL-28* region in the Caucasian "pgt" population. An LD plot of $r^2$ was created in Haploview v4.1,[17] with LD blocks being inferred by the method of Gabriel et al.[18]

Results

Samples were available for a total of 406 treatment naive patients and 426 patients who had not responded to a previous course of treatment with peginterferon alfa-2b (12KD). The analysis of EVR was based on data from 800 patients, including 363 individuals (45%) who achieved an EVR and 437 individuals (55%) who did not achieve an EVR. The analysis of SVR was based on data from 663 patients, including 245 patients (37%) who achieved an SVR and 418 (63%) who did not achieve an SVR. The baseline characteristics of patients included in the analysis of EVR and SVR are shown in TABLE 1.

The univariate logistic regression models of baseline factors showed that HCV genotype ($p=1.50 \times 10^{-25}$), age ($5.80 \times 10^{-18}$), ALT quotient ($p=7.50 \times 10^{-10}$), race ($p=9.20 \times 10^{-7}$), BMI ($p=4.70 \times 10^{-5}$) and histological diagnosis ($p=1.30 \times 10^{-5}$) were significantly associated with EVR, and that HCV genotype ($p=4.30 \times 10^{-27}$), age ($p=5.50 \times 10^{-18}$), ALT quotient ($p=2.10 \times 10^{-8}$), race ($p=2.10 \times 10^{-8}$), histological diagnosis ($p=5.40 \times 10^{-7}$), BMI ($p=7.20 \times 10^{-6}$) and HCV RNA level ($p=0.0022$) were significantly associated with SVR. Of note sex was not significantly associated with either EVR or SVR and HCV RNA level was not significantly associated with SVR.

GWAS results in the overall genotype 1 population

A total of 4 samples analysed for genotype were excluded because of high kinship coefficients (indicating a high degree of relatedness). The analysis of data from genotype 1 patients included 627 patients with known EVR status (215 responders [34.3%] and 412 nonresponders [65.7%]) and 516 patients with known SVR status (128 responders [24.8%] and 388 non-responders [75.2%]).

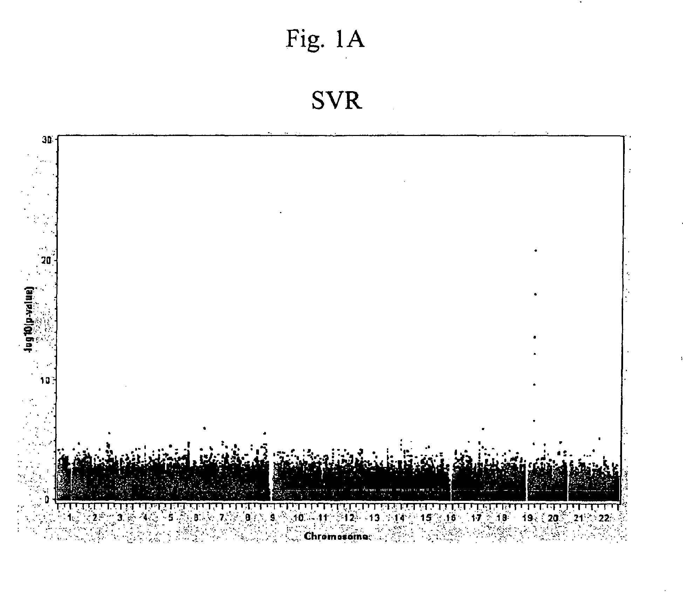

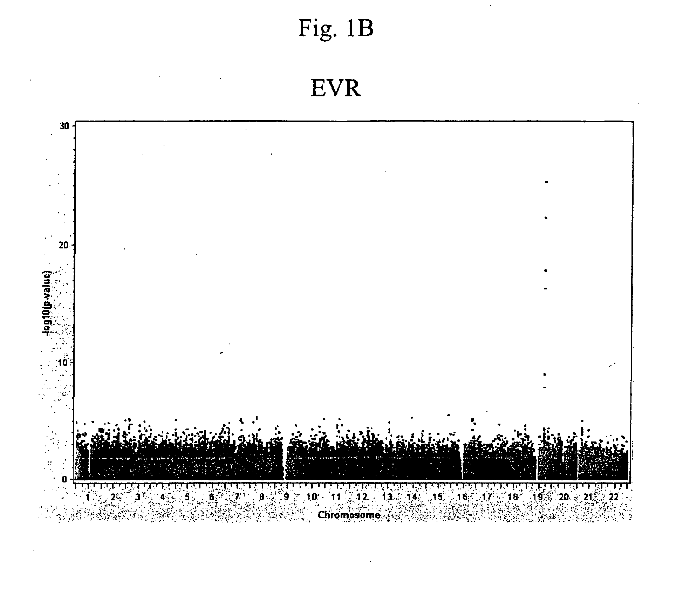

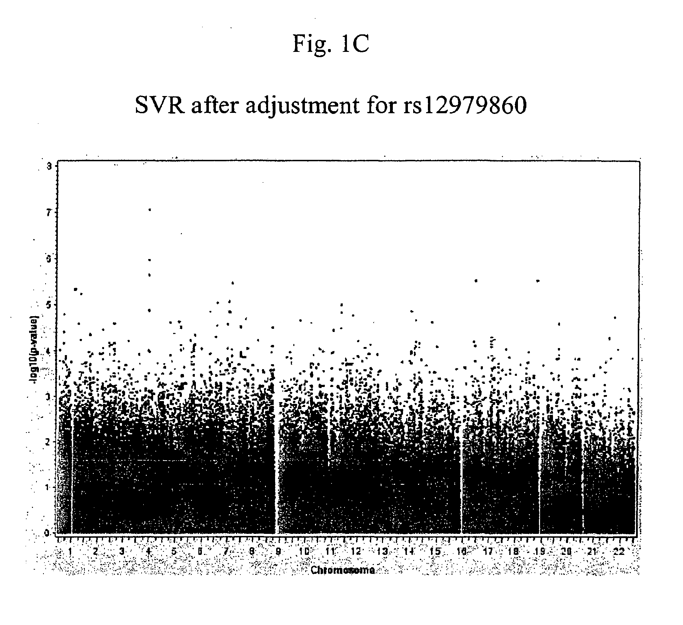

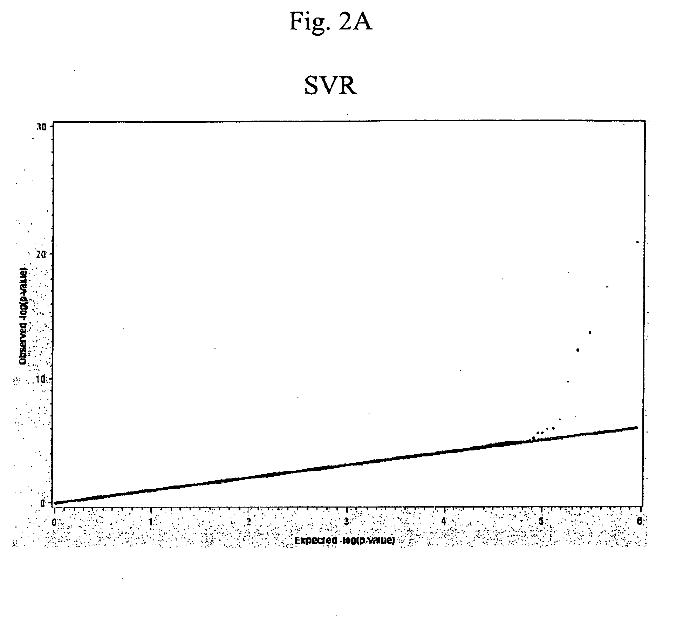

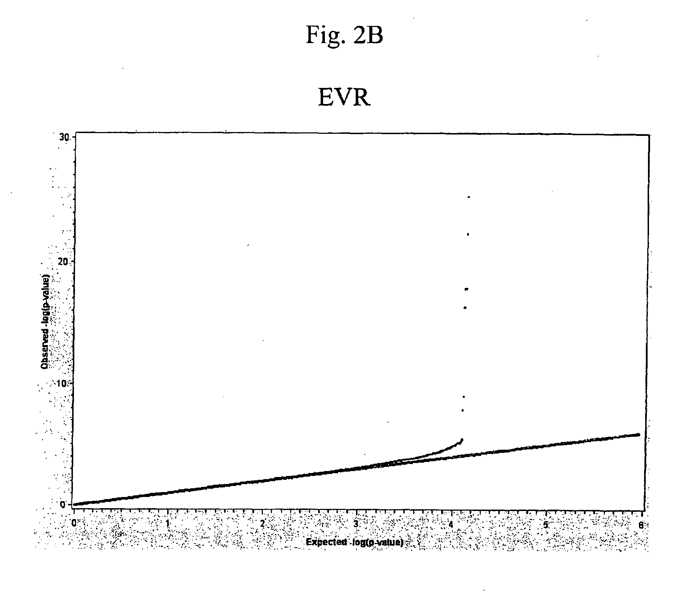

Genome wide association results for SVR and EVR are presented by chromosome in Figure 1. A series of highly significant p values were identified in the IL-28 region on chromosome 19. Quantile-quantile plots showed that the expected and observed p values conform closely with the exception of a few large deviations for the p values associated with chromosome 19 (Figure 2). The logistic regression analysis for SVR and EVR revealed 12 and 19 SNPs, respectively, with $p<10^{-5}$ (TABLE 2).

The top 6 SNPs associated with SVR and EVR, respectively, were identical and fell in the IL-28 region of chromosome 19. The top two SNPs for SVR and EVR were rs12979860 ($p=1.4 \times 10^{-21}$ and $p=5.0 \times 10^{-26}$, respectively)

and rs12980275 (p=5.8x10$^{-18}$ and p=4.9x10$^{-23}$, respectively). Of the remaining six SNPs associated with SVR (TABLE 2), none were associated with EVR, none were located on chromosome 19 and four (rs943897, rs17671102, rs4961441 and rs1892723) may be spurious associations because of the small number of observations in the rare homozygote class (BB <10 individuals). Of the remaining 13 SNPs associated with EVR (TABLE 2), none were associated with SVR, only one (rs4803223) was located on chromosome 19, and two (rs1189800 and rs4975629) may be spurious associations.

Logistic regression analyses of all SNPs with p<10$^{-5}$ for any model of SVR and EVR are shown in TABLE 3 for the overall population and for the subgroup of self-reported Caucasians. When the analyses were repeated after adjustment for the effect of rs12979860 none of the markers were significantly associated with SVR and only one marker was significantly associated with EVR (rs8099917, p=0.0130).

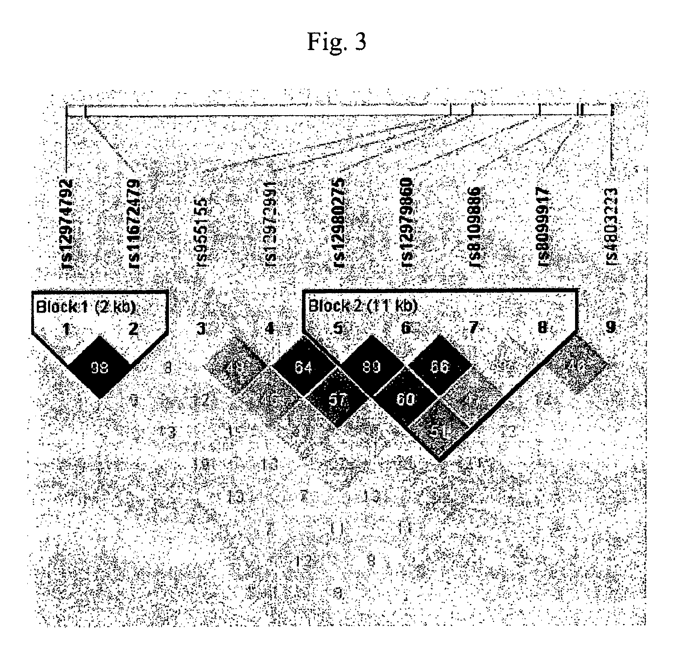

Subsequent investigation of linkage disequilibrium among SNPs in the *IL-28* region presented in TABLE 3 demonstrated one cluster with a high degree of linkage disequilibrium (between rs12979860 and rs12980275, r$^2$=0.98) and a second cluster of SNPs downstream comprised of rs12980275, rs12979860, rs8109886 and rs8099917 (Figure 3).

The genome wide results for SVR after adjustment for rs12979860 are shown in Figure 1c. The results of logistic regression analysis for SVR after adjustment for rs12979860 and inclusion of additional non-responders revealed an additional 10 SNPs with p<10$^{-5}$ (Table 4). Interestingly, three of the five most prominent of which (rs10009948, rs10023606, and rs7673763) are located on chromosome 4. Curiously, these were associated exclusively with SVR, and not with EVR.

<u>Exploratory analysis of rs12979860 in the Caucasian self-reported population</u>

In order to better characterize and understand the association between IL28B, we first explore the association between rs12979860 in Non-genotype 1 in the self-reported Caucasian population. With the same logistic regression model as in the GWAS, a borderline significant association between SVR and the number of rs12979860 C allele is seen (OR=1.88, 95% CI= [0.97; 3.67], p= 0.06) ,at the 5% nominal type 1 error. Similarly, a significant association between EVR and the number of rs12979860 C allele (OR=2.27, 95% CI [ 1.12; 4.70], p=0.02).

In addition, we also describe the association between rs12979860 in the two trials separately. In the naive-treated population, an association between EVR and rs12979860 C allele is seen with an OR of nearly 5 (OR=4.98 95% CI [2.35; 10.53], p=2.6X10$^{-5}$). The association is constantly seen in all the three treatment strata.

In the patient population who failed on pegylated-interferon therapy, no association was found with sustained viral response. However an association with rs12979860 persists when comparing EVR (N=185) and non-EVR (N=154) (OR=1.91 [1.22; 2.96], p=0.003). The association was independent from the viral load at baseline, gender, age, treatment and ALT Quotient.

References

1. Ghany MG, Strader DB, Thomas DL, Seeff LB. Diagnosis, management, and treatment of hepatitis C: An update. Hepatology 2009;49:1335-1374.

2. Manns MP, McHutchison JG, Gordon SC, Rustgi VK, Shiffman M, Reindollar R, Goodman ZD, Koury K, Ling M, Albrecht JK. Peginterferon alfa-2b plus ribavirin compared with interferon alfa-2b plus ribavirin for initial treatment of chronic hepatitis C: a randomised trial. Lancet 2001;358:958-965.

3. Fried MW, Shiffman ML, Reddy KR, Smith C, Marinos G, Goncales FL, Jr., Haussinger D, Diago M, Carosi G, Dhumeaux D, Craxi A, Lin A, Hoffman J, Yu J. Peginterferon alfa-2a plus ribavirin for chronic hepatitis C virus infection. N Eng1 J Med 2002;347:975-982.

4. Hadziyannis SJ, Sette H, Jr., Morgan TR, Balan V, Diago M, Marcellin P, Ramadori G, Bodenheimer H, Jr., Bernstein D, Rizzetto M, Zeuzem S, Pockros PJ, Lin A, Ackrill AM. Peginterferon-alpha2a and ribavirin combination therapy in chronic hepatitis C: a randomized study of treatment duration and ribavirin dose. Ann Intern Med 2004;140:346-355.

5. Conjeevaram HS, Fried MW, Jeffers LJ, Terrault NA, Wiley-Lucas TE, Afdhal N, Brown RS, Belle SH, Hoofnagle JH, Kleiner DE, Howell CD. Peginterferon and ribavirin treatment in African American and Caucasian American patients with hepatitis C genotype 1. Gastroenterology 2006;131:470-477.

6. Dienstag JL, McHutchison JG. American Gastroenterological Association technical review on the management of hepatitis C. Gastroenterology 2006;130:231-264.

7. Missiha S, Heathcote J, Arenovich T, Khan K. Impact of asian race on response to combination therapy with peginterferon alfa-2a and ribavirin in chronic hepatitis C. Am J Gastroenterol 2007;102:2181-2188.

8. Reddy KR, Messinger D, Popescu M, Hadziyannis SJ. Peginterferon alpha-2a (40 kDa) and ribavirin: comparable rates of sustained virological response in sub-sets of older and younger HCV genotype 1 patients. J Viral Hepat 2009;16:724-731.

9. Ferenci P, Fried MW, Shiffman ML, Smith CI, Marinos G, Goncales FL, Jr., Haussinger D, Diago M, Carosi G, Dhumeaux D, Craxi A, Chaneac M, Reddy KR. Predicting sustained virological responses in chronic hepatitis C patients treated with peginterferon alfa-2a (40 KD)/ribavirin. J Hepatol 2005;43:425-433.

10. Martinot-Peignoux M, Maylin S, Moucari R, Ripault MP, Boyer N, Cardoso AC, Giuily N, Castelnau C, Pouteau M, Stem C, Auperin A, Bedossa P, Asselah T, Marcellin P. Virological response at 4 weeks to predict outcome of hepatitis C treatment with pegylated interferon and ribavirin. Antivir Ther 2009;14:501-511.

11. Asselah T, Bieche I, Sabbagh A, Bedossa P, Moreau R, Valla D, Vidaud M, Marcellin P. Gene expression and hepatitis C virus infection. Gut 2009;58:846-858.

12. Ge D, Fellay J, Thompson AJ, Simon JS, Shianna KV, Urban TJ, Heinzen EL, Qiu P, Bertelsen AH, Muir AJ, Sulkowski M, McHutchison JG, Goldstein DB. Genetic variation in IL28B predicts hepatitis C treatment-induced viral clearance. Nature 2009;461:399-401.

13. Suppiah V, Moldovan M, Ahlenstiel G, Berg T, Weltman M, Abate ML, Bassendine M, Spengler U, Dore GJ, Powell E, Riordan S, Sheridan D, Smedile A, Fragomeli V, Muller T, Bahlo M, Stewart GJ, Booth DR, George J. IL28B is associated with response to chronic hepatitis C interferon-alpha and ribavirin therapy. Nat Genet 2009;41:1100-1104.

14. Tanaka Y, Nishida N, Sugiyama M, Kurosaki M, Matsuura K, Sakamoto N, Nakagawa M, Korenaga M, Hino K, Hige S, Ito Y, Mita E, Tanaka E, Mochida S, Murawaki Y, Honda M, Sakai A, Hiasa Y, Nishiguchi S, Koike A, Sakaida I, Imamura M, Ito K, Yano K, Masaki N, Sugauchi F, Izumi N, Tokunaga K, Mizokami M. Genome-wide association of IL28B with response to pegylated interferon-alpha and ribavirin therapy for chronic hepatitis C. Nat Genet 2009;41:1105-1109.

15. Jensen DM, Marcellin P, Freilich B, Andreone P, Di BA, Brandao-Mello CE, Reddy KR, Craxi A, Martin AO, Teuber G, Messinger D, Thommes JA, Tietz A. Re-treatment of patients with chronic hepatitis C who do not respond to peginterferon-alpha2b: a randomized trial. Ann Intern Med 2009;150:528-540.

16. Purcell S, Neale B, Todd-Brown K, Thomas L, Ferreira MA, Bender D, Maller J, Sklar P, de Bakker PI, Daly MJ, Sham PC. PLINK: a tool set for whole-genome association and population-based linkage analyses. Am J Hum Genet 2007;81:559-575.

17. Barrett JC, Fry B, Maller J, Daly MJ. Haploview: analysis and visualization of LD and haplotype maps. Bioinformatics 2005;21:263-265.

18. Gabriel SB, Schaffner SF, Nguyen H, Moore JM, Roy J, Blumenstiel B, Higgins J, Defelice M, Lochner A, Faggart M, Liu-Cordero SN, Rotimi C, Adeyemo A, Cooper R, Ward R, Lander ES, Daly MJ, Altshuler D. The structure of haplotype blocks in the human genome. Science 2002;296:2225-2229.

19. Neumann AU, Lam NP, Dahari H, Gretch DR, Wiley TE, Layden TJ, Perelson AS. Hepatitis C viral dynamics in vivo and the antiviral efficacy of interferon-alpha therapy. Science 1998;282:103-107.

20. Thomas DL, Thio CL, Martin MP, Qi Y, Ge D, O'huigin C, Kidd J, Kidd K, Khakoo SI, Alexander G, Goedert JJ, Kirk GD, Donfield SM, Rosen HR, Tobler LH, Busch MP, McHutchison JG, Goldstein DB, Carrington M. Genetic variation in IL28B and spontaneous clearance of hepatitis C virus. Nature 2009;461:798-801.

21. Rauch A, Kutalik Z, Descombes P, Cai T, Di IJ, Mueller T, Bochud M, Battegay M, Bernasconi E, Borovicka J, Colombo S, Cerny A, Dufour JF, Furrer H, Gunthard HF, Heim M, Hirschel B, Malinverni R, Moradpour D, Mullhaupt B, Witteck A, Beckmann JS, Berg T, Bergmann S, Negro F, Telenti A, Bochud PY. Genetic Variation in IL28B Is Associated With Chronic Hepatitis C and Treatment Failure: A Genome-wide Association Study. Gastroenterology 2010.

22. McCarthy JJ, Li JH, Thompson A, Suchindran S, Lao XQ, Patel K, Tillmann HL, Muir AJ, McHutchison JG. Replicated association between an interleukin-28B gene variant and a sustained response to pegylated interferon and ribavirin [manuscript in press]. Gastroenterology 2010.

23. Montes-Cano M, et al. IL28B genetic variants and hepatitis virus infection by different viral genotypes [manuscript in press]. Hepatology 2010.

24. Marcello T, Grakoui A, Barba-Spaeth G, Machlin ES, Kotenko SV, MacDonald MR, Rice CM. Interferons alpha and lambda inhibit hepatitis C virus replication with distinct signal transduction and gene regulation kinetics. Gastroenterology 2006;131:1887-1898.

25. Robek MD, Boyd BS, Chisari FV. Lambda interferon inhibits hepatitis B and C virus replication. J Virol 2005;79:3851-3854.

26. Siren J, Pirhonen J, Julkunen I, Matikainen S. IFN-alpha regulates TLR-dependent gene expression of IFN-alpha, IFN-beta, IL-28, and IL-29. J Immunol 2005;174:1932-1937.

27. Asselah T, Bieche I, Narguet S, Sabbagh A, Laurendeau I, Ripault MP, Boyer N, Martinot-Peignoux M, Valla D, Vidaud M, Marcellin P. Liver gene expression signature to predict response to pegylated interferon plus ribavirin combination therapy in patients with chronic hepatitis C. Gut 2008;57:516-524.

28. Esteban M. Hepatitis C and evasion of the interferon system: a PKR paradigm. Cell Host Microbe 2009;6:495-497.

29. Asselah T, Benhamou Y, Marcellin P. Protease and polymerase inhibitors for the treatment of hepatitis C. Liver Int 2009;29 Suppl 1:57-67.

30. Foy E, Li K, Wang C, Sumpter R, Jr., Ikeda M, Lemon SM, Gale M, Jr. Regulation of interferon regulatory factor-3 by the hepatitis C virus serine protease. Science 2003;300:1145-1148.

31. Reesink HW, Zeuzem S, Weegink CJ, Forestier N, van VA, van de Wetering de Rooij, McNair L, Purdy S, Kauffman R, Alam J, Jansen PL. Rapid decline of viral RNA in hepatitis C patients treated with VX-950: a phase Ib, placebo-controlled, randomized study. Gastroenterology 2006;131:997-1002.

32. Gane E, Roberts S, Stedman C, Angus P, Ritchie B, Elston R, Ipe D, Morcos P, Najera I, Chu T, Berrey M, Bradford W, Laughlin M, Shulman N, Smith P. Combination therapy with a nucleoside polymerase (R7128) and protease (R7227/ITMN-191) inhibitor in HCV: safety, pharmacokinetics , and virologic results from INFORM-1 [abstract 193]. Hepatology 2009;50 (Supplement):394-5A.

33. Askarieh G, Alsio A, Pugnale P, Negro F, Ferrari C, Neumann AU, Pawlotsky JM, Schalm SW, Zeuzem S, Norkrans G, Westin J, Soderholm J, Hellstrand K, Lagging M. Systemic and intrahepatic interferon-gamma-inducible protein 10 kDa predicts the first-phase decline in hepatitis C virus RNA and overall viral response to therapy in chronic hepatitis C. Hepatology 2009.

34. Lanford RE, Hildebrandt-Eriksen ES, Petri A, Persson R, Lindow M, Munk ME, Kauppinen S, Orum H. Therapeutic silencing of microRNA-122 in primates with chronic hepatitis C virus infection. Science 2010;327:198-201.

TABLE 1

| | | EVR analysis (N=800) | | SVR analysis (N=663) | |
|---|---|---|---|---|---|
| | | No EVR (N=437) | EVR (N=363) | No SVR (N=418) | SVR (N=245) |
| Gender | Female, n (%) | 144 (33.0) | 107 (29.5) | 132 (31.6) | 78 (31.8) |
| | Male, n (%) | 293 (67.0) | 256 (70.5) | 286 (68.4) | 167 (68.2) |
| Mean age $\pm$ SD, years | | 48.14 $\pm$ 8.89 | 41.97 $\pm$ 9.54 | 48.07 $\pm$ 9.03 | 40.87 $\pm$ 9.51 |
| Mean BMI $\pm$ SD, kg/m$^2$ | | 28.12 $\pm$ 5.4 | 26.51 $\pm$ 5.41 | 28.16 $\pm$ 5.21 | 26.15 $\pm$ 5.64 |
| Race/ethnicity, n (%) | | | | | |
| Caucasian | | 392 (89.7) | 288 (79.3) | 369 (88.3) | 196 (80.0) |
| Black | | 39 (8.9) | 10 (2.8) | 37 (8.9) | 5 (2.0) |
| Oriental | | 5 (1.1) | 38 (10.5) | 6 (1.4) | 31 (12.7) |
| Other | | 1 (0.2) | 27 (7.4) | 6 (1.4) | 13 (5.3) |
| Mean ALT quotient $\pm$ SD | | 2.21 $\pm$ 1.48 | 3.09 $\pm$ 2.25 | 2.29 $\pm$ 1.49 | 3.25 $\pm$ 2.5 |
| Histological diagnosis, n (%)[a] | | | | | |
| No cirrhosis | | 324 (74.1) | 316 (87.1) | 307 (73.4) | 222 (90.6) |
| Cirrhosis | | 111 (25.4) | 47 (12.9) | 109 (26.1) | 23 (9.4) |
| HCV genotype, n (%) | | | | | |
| 1 | | 412 (94.3) | 215 (59.2) | 388 (92.8) | 128 (52.2) |
| Non-1 | | 25 (5.7) | 148 (40.8) | 30 (7.2) | 117 (47.8) |
| Serum HCV RNA, IU/mL x 10$^6$ | | 4.8 $\pm$ 5.8 | 6.3 $\pm$ 8.0 | 5.0 $\pm$ 6.0 | 6.2 $\pm$ 8.8 |
| a. Histological diagnosis was missing for 2 EVR/SVR non-responders | | | | | |

TABLE 2

| SNP | Chromosome; gene (location) | SVR | | | | | | EVR | | | | | |
|---|---|---|---|---|---|---|---|---|---|---|---|---|---|
| | | No. of patients | | | Allele (MAF) | p-value | OR;[a]95% CI | No. of patients | | | Allele (MAF) | p-value | OR; [a]95% CI |
| | | AA | AB | BB | | | | AA | AB | BB | | | |
| | | | | | | | | | | | | | |

Results of the logistic regression analysis of SVR and EVR in the overall genotype 1 population showing all SNPs with $p<10^{-5}$ in the primary analysis

| SNP | Chromosome; gene (location) | AA | AB | BB | Allele (MAF) | p-value | OR;[a]95% CI | AA | AB | BB | Allele (MAF) | p-value | OR; [a]95% CI |
|---|---|---|---|---|---|---|---|---|---|---|---|---|---|
| rs12979860 | 19; IL28B (flanking 5' UTR) | 102 | 314 | 100 | G:A (0.5) | $1.4 \times 10^{-21'''}$ | 0.11; 0.07-0.19 | 139 | 371 | 117 | G:A (0.48) | $5.00 \times 10^{-'\circ}$ | 0.15; 0.1-0.23 |
| rs12980275 | 19; IL28B (flanking 3' UTR) | 116 | 317 | 83 | A:G (0.47) | $5.81 \times 10^{-18}$ | 0.15; 0.09-0.24 | 156 | 371 | 100 | A:G (0.46) | $4.90 \times 10^{-23}$ | 0.18; 0.12-0.26 |
| rs8109886 | 19; LOC441849 (flanking 3' UTR) | 169 | 274 | 73 | A:C (0.41 ) | $2.22 \times 10^{-14}$ | 4.77; 3.06-7.44 | 200 | 327 | 100 | A:C (0.42) | $1.55 \times 10^{-18}$ | 4.13; 2.91-5.85 |
| rs8099917 | 19; LOC441849 (flanking 3' UTR) | 219 | 260 | 37 | A:C (0.20) | $5.75 \times 10^{-13}$ | 0.20; 0.12-0.32 | 282 | 302 | 43 | A:C (0.31) | $4.92 \times 10^{-17}$ | 0.23; 0.15-0.33 |
| rs12972991 | 19; IL28B (flanking 3' UTR) | 201 | 274 | 41 | A:C (0.34) | $2.05 \times 10^{-10}$ | 0.26; 0.17-0.41 | 260 | 318 | 49 | A:C (0.33) | $1.45 \times 10^{-18}$ | 0.22; 0.15-0.31 |
| rs955155 | 19; LOC728942 (flanking 5'UTR) | 240 | 233 | 43 | G:A (0.31) | $2.19 \times 10^{-7}$ | 0.36; 0.25-0.53 | 300 | 274 | 53 | G:A (0.3) | $9.77 \times 10^{-10}$ | 0.37; 0.27-0.52 |
| rs9493897 | 6; LOC44261 (flanking 3'UTR) | 404 | 103 | 9 | G:A (0.12) | $1.07 \times 10^{-6}$ | 3.37; 2.06-5.51 | | | | | | |
| rs17671102 | 17; MS12 (flanking 3'UTR) | 443 | 68 | 4 | G:A (0.07) | $1.18 \times 10^{-6}$ | 4.26; 2.36-7.68 | | | | | | |
| rs9864595 | 3; SUMF1 (intron) | 308 | 190 | 18 | A:C (0.22) | $2.49 \times 10^{-6}$ | 2.68; 1.76-4.07 | | | | | | |
| rs4961441 | 9; C9orf39 (intron) | 479 | 34 | 3 | A:C (0.04) | $2.62 \times 10^{-6}$ | 0.00; 0.00-17.25 | | | | | | |
| rs1892723 | 22; PIWIL3 (coding) | 464 | 51 | 1 | A:G (0.05) | $6.72 \times 10^{-6}$ | 4.63; 2.36-9.08 | | | | | | |
| rs1158442 | 14; KCNH5 (flanking 3' UTR) | 153 | 236 | 126 | A:G (0.47) | $9.66 \times 10^{-6}$ | 0.48; 0.35-0.67 | | | | | | |
| rs4803223 | 19; LOC441849 (flanking 5' UTR) | | | | | | | 372 | 226 | 29 | A:G (0.23) | $1.35 \times 10^{-8}$ | 0.36; 0.25-0.53 |

EP 2 589 669 B1

| Results of the logistic regression analysis of SVR and EVR in the overall genotype 1 population showing all SNPs with p<10⁻⁵ in the primary analysis | | | | | | | | | | | | | |
|---|---|---|---|---|---|---|---|---|---|---|---|---|---|
| SNP | Chromosome; gene (location) | SVR | | | | | | EVR | | | | | |
| | | No. of patients | | | Allele (MAF) | p-value | OR;[a]95% CI | No. of patients | | | Allele (MAF) | p-value | OR; [a]95% CI |
| | | AA | AB | BB | | | | AA | AB | BB | | | |
| rs11635085 | 15; C15orf32 (flanking 3'UTR) | | | | | | | 418 | 184 | 24 | A:C (0.19) | $3.38 \times 10^{-6}$ | 0.43; 0.29-0.63 |
| rs2037204 | 8; TACC1 (coding) | | | | | | | 227 | 303 | 97 | A:G (0.4) | $4.84 \times 10^{-6}$ | 1.88; 1.43-2.48 |
| rs189800 | 14; LOC440180 (flanking 5' UTR) | | | | | | | 581 | 42 | 4 | A:G (0.04) | $5.51 \times 10^{-6}$ | 0.05; 0.01-0.39 |
| rs2642940 | 3; LOC643634 (flanking 5' UTR) | | | | | | | 296 | 259 | 72 | A:G (0.32) | $6.70 \times 10^{-6}$ | 0.52; 0.38-0.69 |
| rs5015755 | 7; ZCWPW1 (intron) | | | | | | | 455 | 159 | 13 | C:A (0.15) | $7.03 \times 10^{-6}$ | 2.29; 1.59-3.3 |
| rs2290841 | 10; ADAM12 (intron) | | | | | | | 411 | 191 | 25 | A:G (0.19) | $7.06 \times 1 0^{-6}$ | 0.45; 0.31-0.65 |
| rs4268783 | 16; CNTNAP4 (UTR) | | | | | | | 293 | 264 | 60 | G:A (0.31) | $7.26 \times 10^{-6}$ | 1.92; 1.44-2.56 |
| rs13252897 | 8; TACC1 (intron) | | | | | | | 237 | 298 | 92 | G:A (0.38) | $7.29 \times 10^{-6}$ | 1.87; 1.41-2.46 |
| rs11643145 | 16; CNTNAP4 (intron) | | | | | | | 275 | 276 | 76 | C:A (0.34) | $7.96 \times 10^{-6}$ | 1.87; 1.41-2.46 |
| rs4975629 | 5; SLC6A19 (cds-synon) | | | | | | | 532 | 92 | 3 | G:A (0.08) | $8.36 \times 10^{-6}$ | 0.26; 0.13-0.5 |
| rs3807475 | 7; ZCWPW1 (5' UTR) | | | | | | | 455 | 160 | 12 | G:A (0.15) | $8.74 \times 10^{-6}$ | 2.29; 1.59-3.3 |

EP 2 589 669 B1

13

(continued)

| Results of the logistic regression analysis of SVR and EVR in the overall genotype 1 population showing all SNPs with $p<10^{-5}$ in the primary analysis | | | | | | | | | | | | | |
|---|---|---|---|---|---|---|---|---|---|---|---|---|---|
| SNP | Chromosome; gene (location) | SVR | | | | | | EVR | | | | | |
| | | No. of patients | | | Allele (MAF) | p-value | OR;[a]95% CI | No. of patients | | | Allele (MAF) | p-value | OR; [a]95% CI |
| | | AA | AB | BB | | | | AA | AB | BB | | | |
| rs7597795 | 2; COL6A3 (intron) | | | | | | | 425 | 182 | 20 | A:G (0.18) | $9.10 \times 10^{-6}$ | 0.41; 0.27-0.62 |
| 95% CI = 95% confidence interval; MAF = minor allele frequency; OR = odds ratio; SNP = single nucleotide polymorphism<br>a. An OR >1 indicates that the probability of SVR or EVR increases as the number of copies of the minor allele increases. Conversely, an OR <1 indicates that the probability of SVR or EVR decreases as the number of copies of the minor allele increases. | | | | | | | | | | | | | |

TABLE 3

| SNP | SVR | | | | | | EVR | | | | | |
|---|---|---|---|---|---|---|---|---|---|---|---|---|
| | Primary analysis | | New definition of non-responders | | Adjusted for rs12979860 | | Primary analysis | | New definition of non-responders | | Adjusted for rs12979860 | |
| | All patients | Caucasian | All patients | Caucasian | All patients | Caucasian | All patients | Caucasian | All patients | Caucasian | All patients | Caucasian |
| rs12979860 | $1.4 \times 10^{-21}$ | $1.82 \times 10^{-19}$ | $6.95 \times 10^{-19}$ | $2.09 \times 10^{-17}$ | NA | NA | $5.00 \times 10^{-26}$ | $8.78 \times 10^{-26}$ | $3.13 \times 10^{-16}$ | $3.32 \times 10^{-26}$ | NA | NA |
| rs12980275 | $5.81 \times 10^{-18}s$ | $6.66 \times 10^{-16}$ | $1.33 \times 10^{-15}$ | $1.70 \times 10^{-14}$ | 0.8980 | 0.5867 | $4.90 \times 10^{-23}$ | $7.05 \times 10^{-23}$ | $6.86 \times 10^{-23}$ | $2.54 \times 10^{-23}$ | 0.4770 | 0.6033 |
| rs8109886 | $2.22 \times 10^{-14}$ | $1.45 \times 10^{-14}$ | $1.60 \times 10^{-12}$ | $4.84 \times 10^{-13}$ | 0.9396 | 0.5251 | $1.55 \times 10^{-11}$ | $1.76 \times 10^{-19}$ | $2.77 \times 10^{-18}$ | $1.61 \times 10^{-19}$ | 0.3722 | 0.2354 |
| rs8099917 | $5.75 \times 10^{-13}$ | $5.55 \times 10^{-11}$ | $1.71 \times 10^{-11}$ | $1.70 \times 10^{-10}$ | 0.1238 | 0.1316 | $4.92 \times 10^{-17}$ | $1.89 \times 10^{-15}$ | $2.02 \times 10^{-17}$ | $4.44 \times 10^{-16}$ | 0.0130 | 0.0379 |
| rs12972991 | $2.05 \times 10^{-10}$ | $8.40 \times 10^{-10}$ | $8.76 \times 10^{-9}$ | $1.30 \times 10^{-8}$ | 0.6464 | 0.6463 | $1.45 \times 10^{-18}$ | $7.50 \times 10^{-18}$ | $4.99 \times 10^{-18}$ | $1.44 \times 10^{-11}$ | 0.0449 | 0.0949 |
| rs955155 | $2.19 \times 10^{-7}$ | $6.22 \times 10^{-7}$ | $1.04 \times 10^{-6}$ | $1.44 \times 10^{-6}$ | 0.9677 | 0.9581 | $9.77 \times 10^{-10}$ | $1.08 \times 10^{-9}$ | $1.31 \times 10^{-9}$ | $1.03 \times 10^{-9}$ | 0.6224 | 0.6471 |
| rs4803223 | $2.06 \times 10^{-5}$ | $8.67 \times 10^{-5}$ | $7.38 \times 10^{-5}$ | $8.77 \times 10^{-5}$ | 0.9035 | 0.9058 | $1.35 \times 10^{-8}$ | $1.55 \times 10^{-8}$ | $2.23 \times 10^{-8}$ | $7.14 \times 10^{-9}$ | 0.1650 | 0.1648 |
| rs12974792 | 0.00251 | 0.00196 | 0.00206 | 0.00136 | 0.7742 | 0.6772 | $6.61 \times 10^{-5}$ | $2.72 \times 10^{-5}$ | $2.37 \times 10^{-5}$ | $1.13 \times 10^{-5}$ | 0.3057 | 0.3169 |
| rs11672479 | 0.00117 | 0.00105 | 0.00094 | 0.00073 | 0.6490 | 0.5308 | $4.62 \times 10^{-5}$ | $3.01 \times 10^{-5}$ | $1.56 \times 10^{-5}$ | $1.23 \times 10^{-5}$ | 0.2718 | 0.3068 |

Results of the logistic regression analysis in the overall genotype 1 population showing all SNPs in the *IL28* region with $p<10^{-5}$ for either SVR or EVR in the primary analysis

TABLE 4

Top findings (SNP with p < 10-5) from logistic regression on SVR in the overall genotype-1 population using the supplemental model which includes adjustment for rs12979860 in the population with additional non-responders.

| SNP | Chr | Position | Genea | Locationa | Alleles | p-value | Odds Ratio* | MAF | N AA | N AB | N BB |
|---|---|---|---|---|---|---|---|---|---|---|---|
| rs10009948 | 4 | 88555708 | - | - | A:G | 2.96E-07 | 3.87 (2.3, 6.51) | 0.13 | 401 | 101 | 14 |
| rs6950645 | 7 | 103360672 | RELN | intron | A:G | 9.76E-07 | 0.34 (0.21, 0.54) | 0.27 | 279 | 193 | 41 |
| rs10908429 | 1 | 152977528 | KCNN3 | intron | G:A | 1.49E-06 | 0.35 (0.22, 0.56) | 0.28 | 263 | 214 | 39 |
| rs10023606 | 4 | 88538319 | - | - | A:C | 2.34E-06 | 3.32 (2.02, 5.47) | 0.14 | 382 | 123 | 11 |
| rs7673763 | 4 | 88568046 | NUDT9 | intron | C:A | 3.93E-06 | 3.21 (1.96, 5.27) | 0.14 | 384 | 117 | 15 |
| rs11158442 | 14 | 62198792 | KCNH5 | flanking 3UTR | A:G | 5.37E-06 | 0.44 (0.3, 0.64) | 0.47 | 153 | 236 | 126 |
| rs1552243 | 5 | 125872994 | GRAMD3 | flanking_3UTR | A:G | 5.65E-06 | 0.41 (0.28, 0.62) | 0.43 | 184 | 221 | 111 |
| rs2299219 | 7 | 86255781 | GRM3 | intron | A:G | 6.22E-06 | 0.31 (0.18, 0.53) | 0.2 | 333 | 162 | 21 |
| rs2646130 | 16 | 83510852 | - | - | A:G | 6.78E-06 | 0.34 (0.21, 0.56) | 0.25 | 289 | 197 | 30 |
| rs11953659 | 5 | 125893989 | ALDH7A1 | flanking_3UTR | A:G | 9.99E-06 | 0.43 (0.29, 0.64) | 0.46 | 168 | 226 | 122 |

*OR > 1 indicates that as the number of copies of the minor allele increases, an individual will have an increased probability of response
*OR < 1 indicates that as the number of copies of the minor allele increases, an individual will have a decreased probability of response
a. Missing Gene and Location values were not in the NCBI database

**Claims**

**1.** A method for predicting sustained virological response of a human subject infected with HCV to interferon treatment comprising:

providing a sample from said human subject, detecting the presence of a single nucleotide polymorphism within chromosome 4 and determining that said subject has a high likelihood of sustained virological response to interferon treatment if said single nucleotide polymorphism is present, wherein said single nucleotide polymorphism is a G at rs10023606, having the sequence as depicted in SEQ ID NO. 2.

2. The method of claim 1 wherein said subject is infected with Genotype-1 HCV.

3. The method of claims 1-2 wherein said interferon treatment comprises treatment selected from the group of peginterferon alfa-2a monotherapy, peginterferon alfa-2a with ribavirin, or interferon alfa-2b with ribavirin.

**Patentansprüche**

1. Verfahren zur Vorhersage einer anhaltenden virologischen Antwort eines menschlichen Individuums, das mit HVC infiziert ist, auf eine Interferon-Behandlung, umfassend:

   Bereitstellen einer Probe von dem menschlichen Individuum, Nachweisen des Vorliegens eines Einzelnucleotid-Polymorphismus innerhalb des Chromosoms 4 und Bestimmen, dass das Individuum mit hoher Wahrscheinlichkeit eine anhaltende virologische Antwort auf die Interferon-Behandlung haben wird, wenn der Einzelnucleotid-Polymorphismus vorliegt, wobei der Einzelnucleotid-Polymorphismus ein G an rs10023606 ist mit der Sequenz wie in SEQ ID NO:2 dargestellt.

2. Verfahren nach Anspruch 1, wobei das Individuum mit Genotyp-1-HCV infiziert ist.

3. Verfahren nach den Ansprüchen 1 bis 2, wobei die Interferon-Behandlung eine Behandlung umfasst, die ausgewählt ist aus der Gruppe Peginterferon-alfa-2a-Monotherapie, Peginterferon-alfa-2a mit Ribavirin oder Interferon-alfa-2b mit Ribavirin.

**Revendications**

1. Procédé pour prédire une réponse virologique soutenue d'un sujet humain infecté avec VHC à un traitement par interféron comprenant:

   la fourniture d'un échantillon provenant dudit sujet humain, la détection de la présence d'un polymorphisme d'un seul nucléotide dans le chromosome 4 et la détermination de ce que ledit sujet a une grande probabilité de réponse virologique soutenue à un traitement par interféron si ledit polymorphisme d'un seul nucléotide est présent, où ledit polymorphisme d'un seul nucléotide est un G à rs 10023606 ayant la séquence représentée dans SEQ ID NO: 2.

2. Procédé selon la revendication 1 où ledit sujet est infecté avec VHC de génotype 1.

3. Procédé selon les revendications 1-2 où ledit traitement par interféron comprend un traitement choisi dans le groupe de monothérapie avec le peginterféron alfa-2a, peginterféron alfa-2a avec la ribavirine et interféron alfa-2b avec la ribavirine.

Fig. 1A

SVR

## Fig. 1B

## EVR

## Fig. 1C

## SVR after adjustment for rs12979860

Fig. 2A

SVR

Fig. 2B

EVR

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4897471 A

- US 4695623 A

**Non-patent literature cited in the description**

- Genetic variation in IL28B is associated with chronic hepatitis C and treatment failure: a genome-wide association study. **RAUCH ANDRI et al.** GASTROENTEROLOGY. ELSEVIER, 01 April 2010, vol. 138, 1338-1345
- **GHANY MG ; STRADER DB ; THOMAS DL ; SEEFF LB.** Diagnosis, management, and treatment of hepatitis C: An update. *Hepatology,* 2009, vol. 49, 1335-1374
- **MANNS MP ; MCHUTCHISON JG ; GORDON SC ; RUSTGI VK ; SHIFFMAN M ; REINDOLLAR R ; GOODMAN ZD ; KOURY K ; LING M ; ALBRECHT JK.** Peginterferon alfa-2b plus ribavirin compared with interferon alfa-2b plus ribavirin for initial treatment of chronic hepatitis C: a randomised trial. *Lancet,* vol. 358, 958-965
- **FRIED MW ; SHIFFMAN ML ; REDDY KR ; SMITH C ; MARINOS G ; GONCALES FL, JR. ; HAUSSINGER D ; DIAGO M ; CAROSI G ; DHUMEAUX D.** Peginterferon alfa-2a plus ribavirin for chronic hepatitis C virus infection. *N Eng1 J Med,* 2002, vol. 347, 975-982
- **HADZIYANNIS SJ ; SETTE H, JR. ; MORGAN TR ; BALAN V ; DIAGO M ; MARCELLIN P ; RAMADORI G ; BODENHEIMER H, JR. ; BERNSTEIN D ; RIZZETTO M.** Peginterferon-alpha2a and ribavirin combination therapy in chronic hepatitis C: a randomized study of treatment duration and ribavirin dose. *Ann Intern Med,* 2004, vol. 140, 346-355
- **CONJEEVARAM HS ; FRIED MW ; JEFFERS LJ ; TERRAULT NA ; WILEY-LUCAS TE ; AFDHAL N ; BROWN RS ; BELLE SH ; HOOFNAGLE JH ; KLEINER DE.** Peginterferon and ribavirin treatment in African American and Caucasian American patients with hepatitis C genotype 1. *Gastroenterology,* 2006, vol. 131, 470-477
- **DIENSTAG JL ; MCHUTCHISON JG.** American Gastroenterological Association technical review on the management of hepatitis C. *Gastroenterology,* 2006, vol. 130, 231-264

- **MISSIHA S ; HEATHCOTE J ; ARENOVICH T ; KHAN K.** Impact of asian race on response to combination therapy with peginterferon alfa-2a and ribavirin in chronic hepatitis C. *Am J Gastroenterol,* 2007, vol. 102, 2181-2188
- **REDDY KR ; MESSINGER D ; POPESCU M ; HADZIYANNIS SJ.** Peginterferon alpha-2a (40 kDa) and ribavirin: comparable rates of sustained virological response in sub-sets of older and younger HCV genotype 1 patients. *J Viral Hepat,* 2009, vol. 16, 724-731
- **FERENCI P ; FRIED MW ; SHIFFMAN ML ; SMITH CI ; MARINOS G ; GONCALES FL, JR. ; HAUSSINGER D ; DIAGO M ; CAROSI G ; DHUMEAUX D.** Predicting sustained virological responses in chronic hepatitis C patients treated with peginterferon alfa-2a (40 KD)/ribavirin. *J Hepatol,* 2005, vol. 43, 425-433
- **MARTINOT-PEIGNOUX M ; MAYLIN S ; MOUCARI R ; RIPAULT MP ; BOYER N ; CARDOSO AC ; GIUILY N ; CASTELNAU C ; POUTEAU M ; STEM C.** Virological response at 4 weeks to predict outcome of hepatitis C treatment with pegylated interferon and ribavirin. *Antivir Ther,* 2009, vol. 14, 501-511
- **ASSELAH T ; BIECHE I ; SABBAGH A ; BEDOSSA P ; MOREAU R ; VALLA D ; VIDAUD M ; MARCELLIN P.** Gene expression and hepatitis C virus infection. *Gut,* 2009, vol. 58, 846-858
- **GE D ; FELLAY J ; THOMPSON AJ ; SIMON JS ; SHIANNA KV ; URBAN TJ ; HEINZEN EL ; QIU P ; BERTELSEN AH ; MUIR AJ.** Genetic variation in IL28B predicts hepatitis C treatment-induced viral clearance. *Nature,* 2009, vol. 461, 399-401
- **SUPPIAH V ; MOLDOVAN M ; AHLENSTIEL G ; BERG T ; WELTMAN M ; ABATE ML ; BASSENDINE M ; SPENGLER U ; DORE GJ ; POWELL E.** IL28B is associated with response to chronic hepatitis C interferon-alpha and ribavirin therapy. *Nat Genet,* 2009, vol. 41, 1100-1104

- **TANAKA Y ; NISHIDA N ; SUGIYAMA M ; KURO-SAKI M ; MATSUURA K ; SAKAMOTO N ; NAKA-GAWA M ; KORENAGA M ; HINO K ; HIGE S.** Genome-wide association of IL28B with response to pegylated interferon-alpha and ribavirin therapy for chronic hepatitis C. *Nat Genet,* 2009, vol. 41, 1105-1109
- **JENSEN DM ; MARCELLIN P ; FREILICH B ; ANDREONE P ; DI BA ; BRANDAO-MELLO CE ; REDDY KR ; CRAXI A ; MARTIN AO ; TEUBER G.** Re-treatment of patients with chronic hepatitis C who do not respond to peginterferon-alpha2b: a randomized trial. *Ann Intern Med,* 2009, vol. 150, 528-540
- **PURCELL S ; NEALE B ; TODD-BROWN K ; THOMAS L ; FERREIRA MA ; BENDER D ; MALLER J ; SKLAR P ; DE BAKKER PI ; DALY MJ.** PLINK: a tool set for whole-genome association and population-based linkage analyses. *Am J Hum Genet,* 2007, vol. 81, 559-575
- **BARRETT JC ; FRY B ; MALLER J ; DALY MJ.** Haploview: analysis and visualization of LD and haplotype maps. *Bioinformatics,* 2005, vol. 21, 263-265
- **GABRIEL SB ; SCHAFFNER SF ; NGUYEN H ; MOORE JM ; ROY J ; BLUMENSTIEL B ; HIGGINS J ; DEFELICE M ; LOCHNER A ; FAGGART M.** The structure of haplotype blocks in the human genome. *Science,* 2002, vol. 296, 2225-2229
- **NEUMANN AU ; LAM NP ; DAHARI H ; GRETCH DR ; WILEY TE ; LAYDEN TJ ; PERELSON AS.** Hepatitis C viral dynamics in vivo and the antiviral efficacy of interferon-alpha therapy. *Science,* 1998, vol. 282, 103-107
- **THOMAS DL ; THIO CL ; MARTIN MP ; QI Y ; GE D ; O'HUIGIN C ; KIDD J ; KIDD K ; KHAKOO SI ; ALEXANDER G.** Genetic variation in IL28B and spontaneous clearance of hepatitis C virus. *Nature,* 2009, vol. 461, 798-801
- **RAUCH A ; KUTALIK Z ; DESCOMBES P ; CAI T ; DI IJ ; MUELLER T ; BOCHUD M ; BATTEGAY M ; BERNASCONI E ; BOROVICKA J.** Genetic Variation in IL28B Is Associated With Chronic Hepatitis C and Treatment Failure: A Genome-wide Association Study. *Gastroenterology,* 2010
- **MCCARTHY JJ ; LI JH ; THOMPSON A ; SUCHINDRAN S ; LAO XQ ; PATEL K ; TILLMANN HL ; MUIR AJ ; MCHUTCHISON JG.** Replicated association between an interleukin-28B gene variant and a sustained response to pegylated interferon and ribavirin [manuscript in press. *Gastroenterology,* 2010
- **MONTES-CANO M et al.** IL28B genetic variants and hepatitis virus infection by different viral genotypes [manuscript in press. *Hepatology,* 2010
- **MARCELLO T ; GRAKOUI A ; BARBA-SPAETH G ; MACHLIN ES ; KOTENKO SV ; MACDONALD MR ; RICE CM.** Interferons alpha and lambda inhibit hepatitis C virus replication with distinct signal transduction and gene regulation kinetics. *Gastroenterology,* 2006, vol. 131, 1887-1898
- **ROBEK MD ; BOYD BS ; CHISARI FV.** Lambda interferon inhibits hepatitis B and C virus replication. *J Virol,* 2005, vol. 79, 3851-3854
- **SIREN J ; PIRHONEN J ; JULKUNEN I ; MATIKAINEN S.** IFN-alpha regulates TLR-dependent gene expression of IFN-alpha, IFN-beta, IL-28, and IL-29. *J Immunol,* 2005, vol. 174, 1932-1937
- **ASSELAH T ; BIECHE I ; NARGUET S ; SABBAGH A ; LAURENDEAU I ; RIPAULT MP ; BOYER N ; MARTINOT-PEIGNOUX M ; VALLA D ; VIDAUD M.** Liver gene expression signature to predict response to pegylated interferon plus ribavirin combination therapy in patients with chronic hepatitis C. *Gut,* 2008, vol. 57, 516-524
- **ESTEBAN M.** Hepatitis C and evasion of the interferon system: a PKR paradigm. *Cell Host Microbe,* 2009, vol. 6, 495-497
- **ASSELAH T ; BENHAMOU Y ; MARCELLIN P.** Protease and polymerase inhibitors for the treatment of hepatitis C. *Liver Int,* 2009, vol. 29 (1), 57-67
- **FOY E ; LI K ; WANG C ; SUMPTER R, JR. ; IKEDA M ; LEMON SM ; GALE M, JR.** Regulation of interferon regulatory factor-3 by the hepatitis C virus serine protease. *Science,* 2003, vol. 300, 1145-1148
- **REESINK HW ; ZEUZEM S ; WEEGINK CJ ; FORESTIER N ; VAN VA ; VAN DE WETERING DE ROOIJ ; MCNAIR L ; PURDY S ; KAUFFMAN R ; ALAM J.** Rapid decline of viral RNA in hepatitis C patients treated with VX-950: a phase Ib, placebo-controlled, randomized study. *Gastroenterology,* 2006, vol. 131, 997-1002
- **GANE E ; ROBERTS S ; STEDMAN C ; ANGUS P ; RITCHIE B ; ELSTON R ; IPE D ; MORCOS P ; NAJERA I ; CHU T.** Combination therapy with a nucleoside polymerase (R7128) and protease (R7227/ITMN-191) inhibitor in HCV: safety, pharmacokinetics , and virologic results from INFORM-1 [abstract 193. *Hepatology,* 2009, vol. 50, 394-5A
- **ASKARIEH G ; ALSIO A ; PUGNALE P ; NEGRO F ; FERRARI C ; NEUMANN AU ; PAWLOTSKY JM ; SCHALM SW ; ZEUZEM S ; NORKRANS G.** Systemic and intrahepatic interferon-gamma-inducible protein 10 kDa predicts the first-phase decline in hepatitis C virus RNA and overall viral response to therapy in chronic hepatitis C. *Hepatology,* 2009

• **LANFORD RE ; HILDEBRANDT-ERIKSEN ES ; PETRI A ; PERSSON R ; LINDOW M ; MUNK ME ; KAUPPINEN S ; ORUM H.** Therapeutic silencing of microRNA-122 in primates with chronic hepatitis C virus infection. *Science,* 2010, vol. 327, 198-201

SEQUENCE LISTING

<110> F. Hoffmann-La Roche AG

<120> Single Nucleotide Polymorphisms that predict HCV Treatment Outcomes

<130> 26667 WO

<160> 3

<170> PatentIn version 3.5

<210> SEQ ID NO: 1
<211> 52
<212> DNA
<213> Homo Sapiens

<220>
<221> misc_feature
<222> (27) .. (27)
<223> r indicates a or g

<400> 1
ctgtatcaaa aaaaaaagca agtattraga atccagtacc ctgatcccac ac         52

<210> SEQ ID NO: 2
<211> 52
<212> DNA
<213> Homo Sapiens

<220>
<221> misc_feature
<222> (27).. (27)
<223> k indicates g or t

<400> 1
gtttcaatga taacttccta gaaaatkaat aactatgata ccactttaag aa         52

<210> SEQ ID NO: 3
<211> 52
<212> DNA
<213> Homo Sapiens

<220>
<221> misc_feature
<222> (27) .. (27)
<223> k indicates g or t

<400> 1
ttaaaatttt gctttttaaa acacctkaag tctgtttgct aattttgata aa         52